# EUROPEAN PATENT SPECIFICATION

(11) **EP 4 344 642 B1**
(45) Date of publication and mention of the grant of the patent: **06.08.2025**
(21) Application number: 23197309.0
(22) Date of filing: 14.09.2023
(51) Int. Cl.: A61B 6/00

(54) **COMPUTER-IMPLEMENTED METHOD FOR SETTING X-RAY ACQUISITION PARAMETERS**
COMPUTERIMPLEMENTIERTES VERFAHREN ZUR EINSTELLUNG VON RÖNTGENAUFNAHMEPARAMETERN
PROCÉDÉ MIS EN UVRE PAR ORDINATEUR POUR DÉFINIR DES PARAMÈTRES D'ACQUISITION DE RAYONS X

(30) Priority: 30.09.2022 EP 22199185
(43) Date of publication of application: 03.04.2024
(73) Proprietor: Siemens Healthineers AG, 91301 Forchheim (DE)
(72) Inventor: Eckert, Dominik, 90762 Fürth (DE); Fok, Wai Yan Ryana, 85748 Garching b. München (DE); Hümmer, Christian, 96215 Lichtenfels (DE); Weber, Thomas, 91353 Hausen (DE)
(74) Representative: Siemens Healthineers Patent Attorneys

(56) References cited:
- CN-A- 109 381 212
- US-A1- 2017 311 921

## Description

### Background

In clinical practice, many decisions regarding treatment of a patient are made based on prior information obtained for that patient. Examples of such information are patient size and weight, clinical conditions, previous medical findings, previous therapies, previous surgeries etc. During a follow-up exam, a radiologist uses knowledge of the patient's clinical history to distinguish between new and old findings in the images, making a monitoring of conditions possible in the first place.

In addition to such patient-specific information, a radiographer's knowledge of previously established X-ray acquisition parameters for that patient has proven useful. Such information can be used by a radiographer to set the imaging system in order to obtain consistent image quality during a follow-up exam, to improve the image quality if the previous settings were suboptimal, etc.

A radiographer may have access to previous X-ray images (and the associated acquisition parameters) for a specific patient in a picture archiving and communication system (PACS) database. However, putting such information to use during a step of setting X-ray acquisition parameters can be challenging in a clinical practice due to the lack of standardized software modules and interfaces. Furthermore, information transfer between a PACS and an X-ray imaging system may be unreliable if a wireless internet connection cannot be guaranteed. This can be the case if the imaging system is being used as a mobile scanner, or in an intensive care unit, for example. In any case, since identical follow-up images (same body region and same patient) are generally required only infrequently, prior images and acquisition parameters would be seldom available for a specific patient.

The CN 109 381 212 A discloses a method for automatically determining a scanning region in computed tomography imaging.

It is therefore an object of the invention to provide a way of exploiting available information in a step of setting X-ray acquisition parameters.

Independent of the grammatical term usage, individuals with male, female or other gender identities are included within the term.

According to the invention, the computer-implemented method of setting acquisition parameters for an intended X-ray imaging task comprises steps of: providing a local database with information regarding previously obtained X-ray images and associated acquisition parameters; obtaining a preliminary image for the intended X-ray imaging task; inputting the preliminary image to a machine learning algorithm previously trained to identify one or more X-ray images in the local database that exhibit similarity to, i.e. are visually similar to, the preliminary image; and outputting the acquisition parameters of such an identified X-ray image as proposed acquisition parameters for the intended X-ray imaging task. As used herein, the term "acquisition parameters" for an X-ray imaging task shall be understood to comprise device parameters which can be adjusted by the user, for example a voltage/power setting of an X-ray generator, the geometrical position of an X-ray tube, etc. The acquisition parameters for an imaging task depend on various factors such as clinical procedure, indication, the device itself, etc. The preliminary image shall be understood to be an image showing the relevant body region of the patient in keeping with the intended X-ray imaging task. For example, if the intended imaging task is to obtain an abdominal X-ray, the preliminary image will show the patient's abdomen.

In the inventive method, the closest matching X-ray images and their associated acquisition parameters are identified in the database, and the user can choose the one which best suits the intended imaging task. This gives two advantages over known approaches of setting acquisition parameters: firstly, the proposed acquisition parameters were already used in a prior X-ray imaging task and can be assumed to be more accurate than simply estimated or guessed acquisition parameters; secondly, the radiologist is shown the closest matching image(s) and has the opportunity to make corrections to the proposed acquisition parameters if necessary, for example if the patient position in a closest matching X-ray image differs slightly from the required orientation in the intended imaging task.

An advantage of the inventive method is that it offers a means of reducing the influence of unintentional subjective preferences. For example, one radiographer may tend to focus on radiation dose, another radiographer may tend to focus on image quality. Furthermore, inexperienced personnel may tend to generate images with more significant errors compared to experienced radiographers. Another factor to consider is that a follow-up scan of the patient and same body region may be done by a different radiographer. By offering a radiographer one or more fitting suggestions for the intended imaging task, a more objective approach is assured. An advantage of the inventive computer-implemented method is that it exploits this type of information, previously stored for this purpose in a local or on-system database, to improve subsequent imaging procedures. In other words, the inventive computer-implemented method overcomes the known limitations by using already available information collected from prior examinations to find the best fitting set of acquisition parameters for the intended imaging task.

The invention further describes a data processing system or "providing system" adapted to carry out the inventive computer-implemented method in order to provide, to the user of an X-ray imaging apparatus, acquisition parameters for an intended imaging task. The inventive data processing system is adapted to carry out the inventive method and comprises a dedicated local database with information pertaining to previously obtained X-ray images and associated acquisition parameters; an input interface for obtaining a preliminary image; a machine learning algorithm previously trained to identify one or more X-ray images in the local database that are similar to the preliminary image; and a means of outputting an identified X-ray image and its acquisition parameters to the user of the X-ray imaging apparatus.

The invention further describes an X-ray imaging apparatus comprising an imaging device adapted to capture a preliminary image of a patient body region in preparation for an intended imaging task, and an instance of the inventive data processing system.

The units or modules of the data processing system can be completely or partially realised as software modules running on a processor of the system. A realisation largely in the form of software modules can have the advantage that applications already installed on an existing system can be updated, with relatively little effort, to carry out the inventive method.

The object of the invention is also achieved by a computer program product with a computer program that is directly loadable into the memory of a data processing system, and which comprises program units to perform the steps of the inventive method when the program is executed by the data processing system. In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently-installed carrier can serve to transport and/or to store the executable parts of the computer program product so that these can be read from a processor unit of a data processing system. A processor unit can comprise one or more microprocessors or their equivalents.

Particularly advantageous embodiments and features of the invention are given by the dependent claims, as revealed in the following description.

The terms "X-ray imaging" and "radiography" shall be understood to be synonyms and may be used interchangeably herein. An X-ray image may also be referred to as a radiography image. During an X-ray imaging procedure, it is usual to first take a pre-shot image in order to decide on the correct dose for that patient and that imaging task (body region and orientation), i.e. to adjust the device settings according to that imaging task and that patient, since absorption is determined in part by the patient's body type and size. The pre-shot image can also be referred to as a preliminary image or a preparatory image. The device settings or acquisition parameters can be the radiation dose, duration and imaged area, for example. The subsequent image, taken with the chosen acquisition parameters, can be referred to herein as the "final image" or "main image".

The user of the computer-implemented method can be a practitioner such as radiographer or similar. In the following, it is assumed that the user is familiar with the radiography apparatus.

The local database of radiography images shall be understood to be an "on-site" database, i.e. the data processing system has direct access to the local database. Any image in the local database can be referred to herein as a "database image" or a "stored image". As mentioned above, the local database contains information pertaining to previously obtained X-ray images and their associated acquisition parameters. While it is possible to store an entire radiography image in the local database, in a preferred embodiment of the invention only derived image data - for example a relevant image sub-region - is stored instead, with the advantage of requiring less storage space. In the following, without restricting the invention in any way, the term "image" as used in the context of the local database shall be understood to comprise an entire image, one or more image sub-regions, etc. An image and its acquisition parameters, once stored in the local database, may be referred to as a "data point" in the following.

The local database can comprise data points relating to all body regions. Equally, the local database can be tailored to a specific body region, for example if the imaging apparatus is deployed in a specialty clinic. The local database preferably stores a suitably large collection of previously generated data points. For example, a "large" collection can comprise at least several hundred in the case of an X-ray apparatus that is used for a single purpose (e.g. a mobile X-ray scanner used to generate chest X-ray images only in an intensive care unit); in the case of an X-ray apparatus used to image various body regions, a "large" collection can comprise many thousands of data points, many tens of thousands of data points, or even more.

A preliminary image is preferably obtained using an RGB camera, in which case the preliminary image is an RGB image. Such camera can be mounted at a suitable location, for example on a housing of the medical imaging apparatus that will be used to carry out the intended imaging task.

Alternatively, a preliminary image can preferably be obtained using the imaging apparatus that will be used to carry out the intended imaging task, i.e. an X-ray image in the case of an X-ray imaging apparatus.

Equally, a preliminary image can be obtained using any suitable alternative imaging modality, for example, a depth-map camera a terahertz imaging apparatus, etc.

In a particularly preferred embodiment of the invention, an identified database image is an image in the local database that matches or resembles the preliminary image. Any such image can be referred to herein as an "identified image" to distinguish it from other images in the local database that are not considered to be similar to the preliminary image.

Preferably, the level of similarity between the preliminary image and a database image is determined on the basis of the number of features common to both images. For example, the preliminary image and a database image may be considered "similar" when these two images have at least a minimum number of features in common. At the very least, both preliminary image and database image preferably show the same body region and the relevant body region of the patient is shown in the same orientation. Such features can be determined using one or more suitable feature extraction tools such as segmentation algorithms, pattern matching algorithms, etc. Preferably, other relevant features are also taken into consideration. For example, in order to classify the preliminary image and a database image as "similar", other common or "shared" features such as patient weight, patient age etc., are also taken into consideration. Some shared features can be identified at the outset on the basis of additional information provided by the user. Therefore, in a preferred embodiment of the invention, input to the machine learning algorithm can comprise the patient-related information or features. For example, the user can augment the preliminary image with additional information such as patient weight, patient age, body region, orientation, etc. Such basic features can be used to reduce computational effort by excluding obviously irrelevant database images. If the intended imaging task is to obtain a frontal image of the chest, only chest images in the local database need be considered by the machine learning algorithm.

The machine learning algorithm then compares the preliminary image to the database images to identify the most similar database image(s). Any "shared" features between the preliminary image and a database image can be identified on the basis of a feature extraction procedure. For example, any image stored to the database can undergo a feature extraction procedure, and the extracted features are labelled or otherwise identified. When the machine learning algorithm is invoked by the data processing system, the preliminary image undergoes the same feature extraction procedure. The machine learning algorithm then compares the extracted features to the features of the images in the database, and identifies any database image with a similar set of features. Ideally, an identified image and the preliminary image have a high degree of similarity. This of course will depend on how well the local database is populated with radiography images.

The machine learning algorithm is preferably trained to classify a database image as "similar" to the preliminary image if these two images have at least a minimum number of features in common, i.e. classification of a database image into "similar" or "dissimilar" is done on the basis of a similarity threshold. For example, a set of features might comprise: body region, orientation, patient weight, patient age, etc.

To this end, the machine learning algorithm can be trained in any suitable manner. In particular, supervised training, self-supervised training, semi-supervised training, unsupervised training, reinforcement learning and/or active learning can be used. Furthermore, a technique such as representation learning or "feature learning" can be applied. In particular, the parameters of the machine learning algorithm can be adapted iteratively by several steps of training.

The machine learning algorithm can be realized in any suitable manner, for example as a neural network, a support vector machine, a decision tree and/or a Bayesian network. Equally, the machine learning algorithm can be based on k-means clustering, Q-learning, genetic algorithms and/or association rules. A neural network can comprise a deep neural network, a convolutional neural network, a convolutional deep neural network, an adversarial network, a deep adversarial network, a generative adversarial network, etc.

Training data for the machine learning algorithm can comprise an image as well as image-specific information. The imaging modality and corresponding specifics can be part of the image metadata, for example if the preliminary image is an X-ray pre-shot, the image metadata may include its acquisition parameters. An image can be augmented by additional data such as the clinical protocol step, etc. A training dataset is effectively the same as a dataset that will be used later on when deploying the trained machine learning algorithm.

A similar image in the local database can be found by comparing the preliminary image against the database images. To find the closest match(es), automatic feature extraction can be applied to find the database image(s) with the closest set of features, i.e. the database image with the greatest number of features in common with the preliminary image. In this way, the machine learning algorithm not only allows finds a database image with the same high-level features (e.g. body part, orientation and shape), but - to the user - the found database image will also look similar to the preliminary image.

Acquisition parameters of a "close match" database image can then be used to set up the imaging apparatus, for example to carry out the intended imaging task or to carry out a low-dose pre-shot imaging task. If the user carries out a pre-shot image using the proposed acquisition parameters, the same procedure can be applied with the taken pre-shot image.

In such a workflow, the machine learning algorithm then identifies one or more similar pre-shot images in the local database and the associated X-ray image(s). These X-ray image(s) are be presented to the user. In the case of more than one closest match, the user can select the most appropriate X-ray image. The corresponding acquisition parameters are then used to set up the imaging apparatus for the intended imaging task.

The advantage of using the initial closest match to take a pre-shot image is that further analysis of the pre-shot image can be done to rule out deficiencies (e.g. to ensure that an organ is properly collimated, that the orientation of the X-ray source is correct by analysing the keystone effect of the pre-shot image, etc.).

Preferably, any pre-shot images and any final image are stored, along with their acquisition parameters in the local database. The "final" X-ray image or main acquisition image can be processed in the usual manner. For example, a main acquisition image can be analysed by any suitable image analytics algorithm, for example to calculate gray values, digital exposure index (DEXI) values, the direct radiation area in the image, the signal-to-noise ratio, etc. The results can then be stored along with the main acquisition image in the local database. By storing the pre-shot and main acquisition images to the local database in this way, the local database can be populated further over time, thereby improving the accuracy of the machine learning algorithm.

An entry of the local database can be augmented by the radiographer's quality assessment to assist in future acquisition parameter proposals. In this way, the radiographer's implicit knowledge of how to choose "good" acquisition parameters is also made accessible to the machine learning algorithm. For example, a quality assessment of the closest match returned by the machine learning algorithm can be given on a scale between "very good" and "very poor". Such an evaluation can assist the machine learning algorithm to improve over time.

In a preferred embodiment of the invention, only anonymized data is stored in the local database of images. Any patient-specific information is stored in a separate database, for example the PACS system of the clinic, which can only be accessed by authorized users. This separates sensitive patient-related data from the anonymized information in the local database used. The advantage of storing information in separate databases is that the local database can be made sharable (internally and/or also externally), since it does not contain any sensitive patient data.

The local database can be stored remotely, for example in the cloud, if access from different locations is to be allowed. Alternatively, the local database can be stored directly in the imaging apparatus system. This can be of particular advantage in the case of mobile imaging systems, which may not always have internet access. For example, an imaging system can build up its own local database over time, or can download an already populated local database from the cloud. Equally, any imaging apparatus can augment its local database using images provided by users at other locations.

An advantage of locally storing the local database is that it is relatively easy to detect differences between a follow-up image and its predecessor. For example, images of a patient can be taken automatically at intervals in an ICU environment, and an alarm is only issued if the newest image differs from the previous image (e.g. when the patient's condition gets worse), ignoring findings that have not changed since the last exam.

The inventive computer-implemented method can be put to use in a number of ways. For example, with the information described above, a self-calibrating imaging system could be realized. In an exemplary workflow, an optical camera or other suitable imaging modality is used to capture a preliminary image, which is input to the data processing system. The machine learning algorithm identifies the closest match in the local database of X-ray images and outputs the acquisition parameters that were used to generate that database image. Using the proposed acquisition parameters, the user sets up the X-ray imaging apparatus to perform the intended imaging task.

Other objects and features of the present invention will become apparent from the following detailed descriptions considered in conjunction with the accompanying drawings. It is to be understood, however, that the drawings are designed solely for the purposes of illustration and not as a definition of the limits of the invention.
Figure 1 shows a schematic representation of an embodiment of the inventive X-ray imaging apparatus;
Figure 2 shows is a block diagram of an embodiment of the inventive data processing apparatus;
Figure 3 shows an exemplary flow-chart of the inventive method;
Figure 4 illustrates a training stage for the machine learning algorithm of the inventive data processing system;
Figure 5 shows a flow-chart to illustrate a prior art imaging procedure;
Figure 6 shows a schematic representation of a neural network as might be deployed in the inventive data processing apparatus.

In the diagrams, like numbers refer to like objects throughout. Objects in the diagrams are not necessarily drawn to scale.

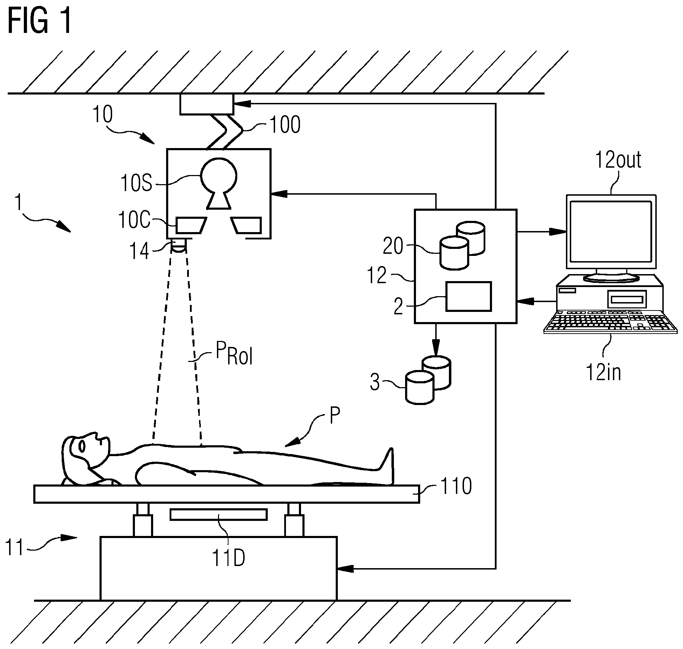

Figure 1 shows an exemplary embodiment of an embodiment of the inventive X-ray apparatus 1 or radiography system 1. The radiography system 1 comprises an X-ray source unit 10, in this case a ceiling-mounted unit which can be moved vertically by an articulated ceiling mount 100. The X-ray source unit 10 encloses an X-ray source 10S and a collimator unit 10C. In this exemplary embodiment of the inventive radiography system 1, a patient positioning device 11 comprises a table 110 which can be raised or lowered as required. Here, a patient P is shown lying on the table 110. The diagram also shows an X-ray detector unit 11D in place below the table 110 and directly underneath the X-ray source 10S. Of course, the imaging apparatus can be realised to deploy a wall-mounted X-ray source unit and a vertical detector.

The radiography system 1 also comprises a control unit 12 for controlling the X-ray source 10S, i.e. the control unit 12 can set the X-ray dose and duration by means of suitable control signals. The control unit 12 can also be configured to actuate the ceiling mount 100 and patient positioning device 11, as well as the collimator 10C, in order to define the radiated area of the patient P. The control unit 12 has an input unit 12ᵢₙ (e.g. a keyboard, touch screen or similar) and an output unit 12ₒᵤₜ (e.g. a monitor or any other kind of display).

The drawing also indicates an instance of the inventive data processing system 2 installed in the control unit 12. The data processing system 2 has access to a local database 20, which can be stored directly in the control unit 12 as indicated here, or which can be stored locally in the clinic facility.

As explained above, a preliminary image can be made using the imaging apparatus itself (in that case the preliminary image is an X-ray pre-shot), or using an alternative imaging modality. Here, the radiography system is also equipped with an RGB camera 14 which can be used to capture a preliminary image of the region of interest P_{RoI} as indicated here. Information collected during imaging apparatus control might be used as input for a machine learning model during a training and inference stage. For example, in an on-device reinforcement learning procedure, a machine learning algorithm could be trained on the same system 1 in which it will be deployed, based on radiographer feedback.

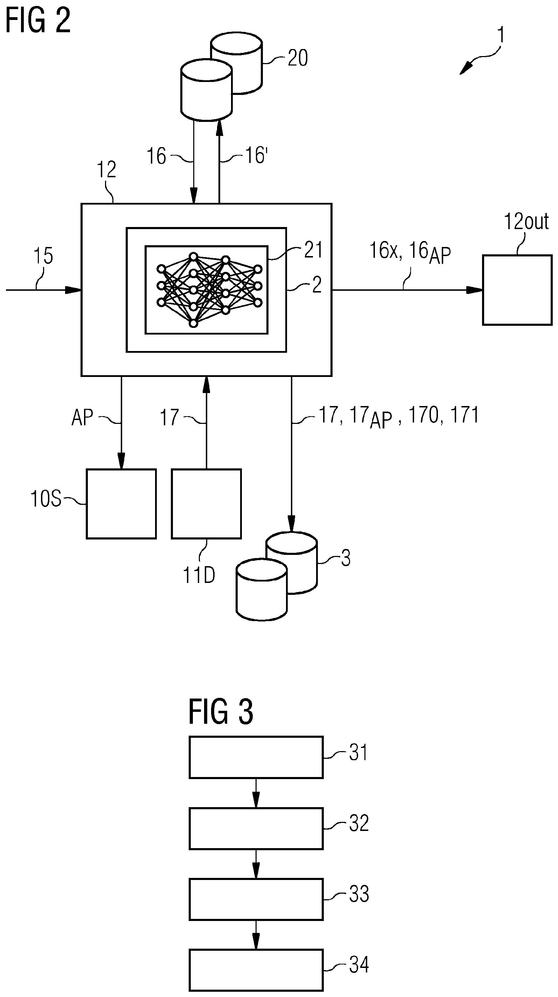

Figure 2 shows a block diagram of an embodiment of the inventive data processing apparatus 2 as might be used in the radiography system 1 of Figure 1.

A preliminary image 15 of the region of interest P_{RoI} of the patient is passed to the data processing system 2 installed on a processor of the control unit 12. The data processing system 2 comprises the trained machine learning algorithm 21 which has access to the local database 20 and its collection of data points. A database entry or data point 16, as explained above, comprises an image 16_{X} and corresponding acquisition parameters 16_{AP}. Any matching or similar images 16_{X} are output to the user, along with their acquisition parameters 16_{AP}. After selecting the best match, the corresponding acquisition parameters AP are used to set up the X-ray source 10S (as well as the patient positioning system and ceiling mount, as appropriate). The main image 17 is then captured by the detector 11D and transmitted to the control unit 12. The control unit 12 can store anonymized main image data and the main image acquisition parameters, along with an image quality assessment given by the user, as a new data point 16' in the local database 20.

The control unit 12 can store the entire image 17 to a PACS database 3. Furthermore, the control unit 12 can include modules that analyse the main image 17 to determine image information 171 such as gray values; the radiated area; a signal-to-noise ratio, etc. The control unit 12 can store such image information 171 to the PACS database 3 along with patient-specific information 170.

Figure 3 shows an exemplary flow-chart of the inventive method. In a first step 31, the user ensures that the patient is positioned in an essentially appropriate position for the intended X-ray imaging task. This basic check can be referred to as a "sanity" check and ensures that the source is directed at the detector and also at the body part to be examined. The user then obtains a preliminary image 15 of the relevant body region of the patient, for example by actuating a camera to capture an RGB image of the region of interest. The preliminary image 15 is then passed to the data processing system in step 32. In step 33, the machine learning algorithm of the data processing system identifies one or more similar images in the local database 20. The results - i.e. any "close match" image 16_{X} and its corresponding acquisition parameters 16_{AP} - are shown to the user, for example in the monitor 12ₒᵤₜ of the control unit 12. The user picks the best match, and issues appropriate commands to apply the corresponding acquisition parameters to the imaging apparatus 1. For example, the selected acquisition parameters can comprise the dose strength and duration for the X-ray source 10S, settings for the collimator 10C, as well as the positions of the table 110 and ceiling mount 100. With suitable acquisition parameters identified in this manner, the intended imaging task is performed in step 34. This generally comprises a step of generating a pre-shot image and then generating the main image. However, with highly accurate proposed acquisition parameters, a pre-shot image may be omitted, so that only the main image is generated in step 34.

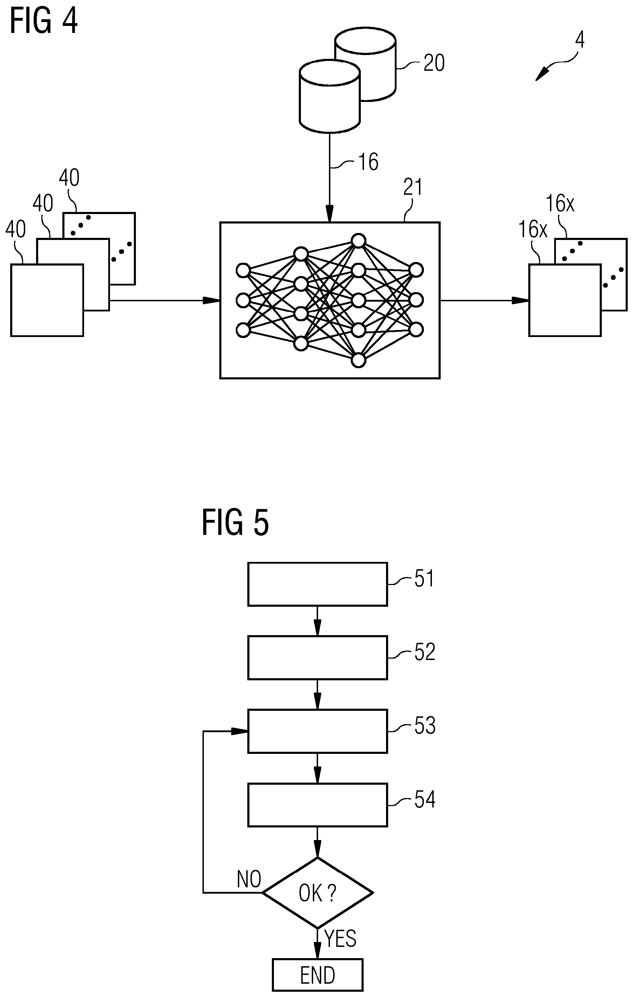

Figure 4 illustrates a training stage 4 for the machine learning algorithm of the inventive data processing system. Each training dataset 40 comprises an image (an RGB image, a depth-map, an X-ray pre-shot, etc.) as well as relevant patient-specific information (height, weight, gender, ethnic group etc.). The imaging modality and corresponding specifics can be part of the image metadata, for example if the input image is an X-ray pre-shot, the image metadata may include its acquisition parameters.

The machine learning algorithm 21 has access to a collection of data points 16 (image or image-derived data along with the corresponding acquisition parameters) stored in a local database 20. The images in the image database 20 are radiography images (i.e. X-ray pre-shot images, X-ray main images) of various body regions and/or for various different patient morphologies.

The machine learning algorithm 21 is trained using a suitable learning approach - unsupervised, self-supervised, supervised, semi-supervised - to identify one or more radiography images 16_{X} or "closest matches" from the local database 20 that exhibit evident similarity to a training dataset image. For example, this can be done by performing feature extraction on a training dataset image and on each image of the local database, and comparing the extracted features. Comparison of any derived metric may be applied, and any candidate database image might be assigned a similarity score with respect to the current training dataset image, so that candidate images 16_{X} with the highest similarity scores are identified. The ground truth for the machine learning algorithm can be that a "similar" image must at least show the same body region for a patient of similar morphology (height and weight).

The choice of ground truth depends on the training procedure, i.e. whether training is unsupervised, supervised, etc. For example, in a supervised training procedure, a two-step approach may be applied. In a first step, relevant images for a body region, morphology and patient information (e.g., age, gender) are selected that contain example cases with various acquisition parameters. In a second step, the machine learning algorithm is trained in a supervised manner to classify which of the selected images matches the current acquisition parameters.

In a supervised training procedure, backward-propagation can be applied to enable the machine learning algorithm to infer the ground truth, in this case to infer whether a database image is a close match for a training dataset image.

Figure 5 illustrates a prior art approach. Here, the acquisition parameters for a pre-shot X-ray image are estimated in step 51. In step 52, the pre-shot X-ray image is taken with the initial estimated acquisition parameters. In step 53, the image is assessed by the radiographer, who then chooses the acquisition parameters for use in step 54 to take the main X-ray image for the intended imaging task. If the main image is not satisfactory on account of sub-optimal acquisition parameters, steps 53, 54 are repeated.

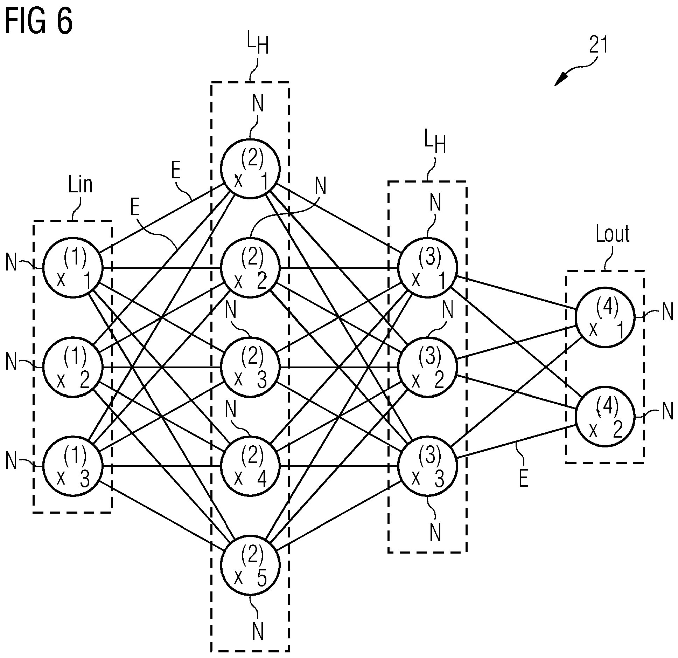

Figure 6 is a highly simplified schematic of an artificial neural network 21 as may be used as the machine learning algorithm of the inventive data processing system 2. The artificial neural network 21 comprises nodes N arranged in layers L, and edges E that form direct connections between nodes of adjacent layers. For clarity, the diagram only shows a few nodes and a few layers, and the skilled person will appreciate that an artificial neural network may comprise many nodes and many layers. The embodiment shown here has an input layer Lᵢₙ, an output layer Lₒᵤₜ, and hidden layers L_{H}. In general, the number of hidden layers L_{H} can be chosen arbitrarily. The number of nodes N within the input layer Lᵢₙ usually relates to the number of input values of the neural network, and the number of nodes N within the output layer Lₒᵤₜ usually relates to the number of output values of the neural network.

A convolutional neural network can be trained based on the backpropagation algorithm. To prevent overfitting, methods of regularization can be used, e.g. dropout of nodes, stochastic pooling, use of artificial data, weight decay based on the L1 or the L2 norm, or max norm constraints, as will be known to the skilled person.

Although the invention has been further illustrated in detail by the preferred embodiments, the invention is not limited by the disclosed examples and other variations may be derived therefrom by those skilled in the art without departing from the scope of protection of the invention.

## Claims

1. A computer-implemented method of setting acquisition parameters (AP) for an intended X-ray imaging task (34), which method comprises
- providing a local database (20) with information regarding X-ray images and associated acquisition parameters;
- obtaining a preliminary image (15);
- inputting the preliminary image (15) to a machine learning algorithm (21) previously trained to identify one or more X-ray images (16_{X}) in the image database (20) that exhibit similarity to the preliminary image (15); and
- outputting the acquisition parameters (16_{AP}) of an identified X-ray image (16_{X}) as proposed acquisition parameters (AP) for the X-ray imaging task (34).

2. A computer-implemented method according to the preceding claim, wherein an identified X-ray image (16_{X}) is the image that most closely matches the preliminary image (15).

3. A computer-implemented method according to any of the preceding claims, wherein similarity between the preliminary image (15) and a database image (16_{X}) is determined on the basis of the number of features common to both images (15, 16_{X}).

4. A computer-implemented method according to any of the preceding claims, wherein the preliminary image (15) is an X-ray image obtained using an X-ray imaging apparatus (1), more preferably an RGB image captured using a camera (14).

5. A computer-implemented method according to any of the preceding claims, comprising a step of augmenting the image database (20) with an X-ray image obtained using the proposed acquisition parameters (AP).

6. A method of performing X-ray imaging, which method comprises the steps of
- obtaining a preliminary image (15) of the body region of a patient (P) to be imaged in an intended X-ray imaging task (34);
- processing the preliminary image (15) using the computer-implemented method according to any of claims 1 to 5 and receiving the proposed acquisition parameters (AP); and
- preforming the intended X-ray imaging task (34) on the basis of the proposed acquisition parameters (AP) to obtain a main image (17).

7. A method according to the preceding claim, comprising a step of analysing the main image (17) to determine image information (171) comprising any of: gray values; radiated area; signal-to-noise ratio.

8. A method according to the preceding claim, comprising a subsequent step of augmenting a separate database (30) with the main image (17) and any of: patient-specific information (170) and/or the image information (171) and/or an image quality assessment (172).

9. A data processing system (2) adapted to carry out the method according to any of claims 1 to 5, which data processing system (2) comprises
- access to a local database (20) of X-ray images and associated acquisition parameters;
- a means (14) of obtaining a preliminary image (15); and
- a machine learning algorithm (21) previously trained to identify one or more X-ray images (16_{X}) in the local database (20) that are similar to the preliminary image (15); and
- a means of outputting an identified X-ray image (16_{X}) and its acquisition parameters (16_{AP}) to a user.

10. A data processing system according to the preceding claim, wherein the local database (20) is shared with a number of further data processing systems.

11. A data processing system according to claim 9 or claim 10, configured to augment the local database (20) with an image (17) taken using the proposed acquisition parameters (16_{AP}) .

12. An X-ray imaging apparatus (1) comprising
- an X-ray source (10S) and an X-ray detector (11D);
- an imaging device (14, 10) adapted to capture a preliminary image (15) of the body region of a patient (P) to be imaged in an intended X-ray imaging task (34);
- a control unit (12) adapted to control at least the X-ray source (10S) and to receive images (17) from the X-ray detector (11D); and
- a data processing system (2) according to any of claims 9 to 11 adapted to receive the preliminary image (15) and to propose acquisition parameters (16_{AP}) on the basis of the preliminary image (15).

13. An X-ray imaging apparatus according to the preceding claim, wherein the imaging device (14) is any of: an RGB camera (14), the X-ray apparatus (1), a terahertz imaging device, a depth-field camera.

14. A computer program product comprising instructions which, when the program is executed by a data processing system (2) according to any of claims 9 to 11, cause the data processing system (2) to carry out the steps of the method according to any of claims 1 to 5.

15. A computer-readable data carrier having stored thereon the computer program product of claim 14.

## Patentansprüche

1. Computerimplementiertes Verfahren zum Einstellen von Erfassungsparametern (AP) für eine beabsichtigte Röntgenbildgebungsaufgabe (34), wobei das Verfahren Folgendes umfasst:
- Bereitstellen einer lokalen Datenbank (20) mit Informationen hinsichtlich Röntgenbildern und zugeordneten Erfassungsparametern;
- Erhalten eines vorläufigen Bilds (15);
- Eingeben des vorläufigen Bilds (15) zu einem Algorithmus (21) für maschinelles Lernen, der zuvor trainiert worden ist, um ein oder mehrere Röntgenbilder (16X) in der Bilddatenbank (20) zu identifizieren, die eine Ähnlichkeit zum vorläufigen Bild (15) aufweisen; und
- Ausgeben der Erfassungsparameter (16AP) eines identifizierten Röntgenbilds (16X) als vorgeschlagene Erfassungsparameter (AP) für die Röntgenbildgebungsaufgabe (34).

2. Computerimplementiertes Verfahren nach dem vorhergehenden Anspruch, wobei ein identifiziertes Röntgenbild (16X) das Bild ist, das mit dem vorläufigen Bild (15) am genauesten übereinstimmt.

3. Computerimplementiertes Verfahren nach einem der vorhergehenden Ansprüche, wobei die Ähnlichkeit zwischen dem vorläufigen Bild (15) und einem Datenbankbild (16X) auf der Grundlage der Anzahl von Merkmalen bestimmt wird, die beide Bilder (15, 16X) gemeinsam haben.

4. Computerimplementiertes Verfahren nach einem der vorhergehenden Ansprüche, wobei das vorläufige Bild (15) ein Röntgenbild ist, das unter Verwendung einer Röntgenbildgebungsvorrichtung (1) erhalten wurde, und stärker bevorzugt ein RGB-Bild ist, das unter Verwendung einer Kamera (14) aufgenommen wurde.

5. Computerimplementiertes Verfahren nach einem der vorhergehenden Ansprüche, das einen Schritt des Erweiterns der Bilddatenbank (20) mit einem Röntgenbild umfasst, das unter Verwendung der vorgeschlagenen Erfassungsparameter (AP) erhalten wurde.

6. Verfahren zum Durchführen von Röntgenbildgebung, wobei das Verfahren die folgenden Schritte umfasst,
- Erhalten eines vorläufigen Bilds (15) des Körperbereichs eines Patienten (P), der in einer beabsichtigten Röntgenbildgebungsaufgabe (34) abgebildet werden soll;
- Verarbeiten des vorläufigen Bilds (15) unter Verwendung des computerimplementierten Verfahrens nach einem der Ansprüche 1 bis 5 und Empfangen der vorgeschlagenen Erfassungsparameter (AP) und
- Vorformen der beabsichtigten Röntgenbildgebungsaufgabe (34) auf der Grundlage der vorgeschlagenen Erfassungsparameter (AP), um ein Hauptbild (17) zu erhalten.

7. Verfahren nach dem vorhergehenden Anspruch, das einen Schritt des Analysierens des Hauptbilds (17) umfasst, um Bildinformationen (171) zu bestimmen, die beliebige der Folgenden umfassen: Grauwerte; einen bestrahlten Bereich; ein Signal/Rausch-Verhältnis.

8. Verfahren nach dem vorhergehenden Anspruch, das einen nachfolgenden Schritt des Erweiterns einer getrennten Datenbank (30) mit dem Hauptbild (17) und beliebige der Folgenden umfasst: patientenspezifische Informationen (170) und/oder die Bildinformationen (171) und/oder eine Bildqualitätsbeurteilung (172).

9. Datenverarbeitungssystem (2), das ausgelegt ist, das Verfahren nach einem der Ansprüche 1 bis 5 auszuführen, wobei das Datenverarbeitungssystem (2) Folgendes umfasst:
- Zugriff auf eine lokale Datenbank (20) von Röntgenbildern und zugeordneten Erfassungsparametern;
- ein Mittel (14) zum Erhalten eines vorläufigen Bilds (15) und
- einen Algorithmus (21) für maschinelles Lernen, der zuvor trainiert worden ist, um ein oder mehrere Röntgenbilder (16X) in der lokalen Datenbank (20) zu identifizieren, die dem vorläufigen Bild (15) ähnlich sind; und
- ein Mittel zum Ausgeben eines identifizierten Röntgenbilds (16X) und seiner Erfassungsparameter (16AP) zu einem Anwender.

10. Datenverarbeitungssystem nach dem vorhergehenden Anspruch, wobei die lokale Datenbank (20) mit mehreren weiteren Datenverarbeitungssystemen gemeinsam verwendet wird.

11. Datenverarbeitungssystem nach Anspruch 9 oder Anspruch 10, das dazu ausgelegt ist, die lokale Datenbank (20) mit einem Bild (17) zu erweitern, das unter Verwendung der vorgeschlagenen Erfassungsparameter (16AP) aufgenommen wurde.

12. Röntgenbildgebungsvorrichtung (1), die Folgendes umfasst:
- eine Röntgenstrahlquelle (10S) und einen Röntgendetektor (11D);
- eine Bildgebungseinrichtung (14, 10), die ausgelegt ist, ein vorläufiges Bild (15) des Körperbereichs eines Patienten (P) aufzunehmen, der in einer beabsichtigten Röntgenbildgebungsaufgabe (34) abgebildet werden soll;
- eine Steuereinheit (12), die ausgelegt ist, mindestens die Röntgenstrahlquelle (10S) zu steuern und Bilder (17) vom Röntgendetektor (11D) zu empfangen; und
- ein Datenverarbeitungssystem (2) nach einem der Ansprüche 9 bis 11, das ausgelegt ist, das vorläufige Bild (15) zu empfangen und Erfassungsparameter (16AP) auf der Grundlage des vorläufigen Bilds (15) vorzuschlagen.

13. Röntgenbildgebungsvorrichtung nach dem vorhergehenden Anspruch, wobei die Bildgebungseinrichtung (14) eine beliebige der Folgenden ist: eine RGB-Kamera (14), die Röntgenvorrichtung (1), eine Terahertzbildgebungseinrichtung, eine Tiefenfeldkamera.

14. Computerprogrammprodukt, das Befehle enthält, die, wenn das Programm durch ein Datenverarbeitungssystem (2) nach einem der Ansprüche 9 bis 11 ausgeführt wird, bewirken, dass das Datenverarbeitungssystem (2) die Schritte des Verfahrens nach einem der Ansprüche 1 bis 5 ausführt.

15. Computerlesbarer Datenträger, in dem das Computerprogrammprodukt nach Anspruch 14 gespeichert ist.

## Revendications

1. Un procédé mis en œuvre par ordinateur de définition de paramètres (AP) d'acquisition pour une tâche (34) envisagée d'imagerie par rayons X, lequel procédé comprend
- se procurer une base (20) locale de données ayant des informations concernant des images aux rayons X et des paramètres d'acquisition associés ;
- obtenir une image (15) préliminaire ;
- entrer l'image (15) préliminaire dans un algorithme (21) d'apprentissage automatique entraîné au préalable à identifier une ou plusieurs images (16_{X}) aux rayons X dans la base (20) de données d'image, qui présentent de la similarité à l'image (15) préliminaire ;
et
- sortir les paramètres (16_{AP}) d'acquisition d'une image (16_{X}) aux rayons X identifiée comme paramètres (AP) d'acquisition proposés pour la tâche (34) d'imagerie aux rayons X.

2. Un procédé mis en œuvre par ordinateur suivant la revendication précédente, dans lequel une image (16_{X}) aux rayons X identifiée est l'image, qui correspond le plus étroitement à l'image (15) préliminaire.

3. Un procédé mis en œuvre par ordinateur suivant l'une quelconque des revendications précédentes, dans lequel de la similarité entre une image (15) préliminaire et une image (16_{X}) de la base de données est déterminée sur la base du nombre de caractéristiques communes aux deux images (15, 16x).

4. Un procédé mis en œuvre par ordinateur suivant l'une quelconque des revendications précédentes, dans lequel l'image (15) préliminaire est une image aux rayons X obtenue en utilisant une installation (1) d'imagerie par rayons X, d'une manière plus préférée une image RGB captée en utilisant un appareil (14) photographique.

5. Un procédé mis en œuvre par ordinateur suivant l'une quelconque des revendications précédentes, comprenant un stade d'augmentation de la base (20) de données d'image par une image aux rayons X obtenue en utilisant les paramètres (AP) d'acquisition proposés.

6. Un procédé pour effectuer une imagerie par rayons X, lequel procédé comprend les stades de
- obtenir une image (15) préliminaire de la région du corps d'un patient (P), dont on doit prendre une image dans une tâche (34) envisagée d'imagerie par rayons X ;
- traiter l'image (15) préliminaire en utilisant le procédé mis en œuvre par ordinateur suivant l'une quelconque des revendications 1 à 5 et recevoir les paramètres (AP) d'acquisition proposés ; et
- préformer la tâche (34) envisagée d'imagerie par rayons X sur la base des paramètres (AP) d'acquisition proposés pour obtenir une image (17) principale.

7. Un procédé suivant la revendication précédente, comprenant un stade d'analyse de l'image (17) principale pour déterminer des informations (171) d'image, comprenant l'une quelconque de : des valeurs de gris ; une zone exposée ; un rapport de signal à bruit.

8. Un procédé suivant la revendication précédente, comprenant un stade venant ensuite d'augmentation d'une base (30) de données distincte par l'image (17) principale et l'une quelconque de : une information (170) spécifique au patient et/ou l'information (171) d'image et/ou une estimation (172) de la qualité de l'image.

9. Un système (2) de traitement de données propre à effectuer le procédé suivant l'une quelconque des revendications 1 à 5, lequel système (2) de traitement de données comprend
- un accès à une base (20) locale de données d'images aux rayons X et à des paramètres d'acquisition associés ;
- un moyen (14) d'obtention d'une image (15) préliminaire ; et
- un algorithme (21) d'apprentissage automatique entraîné au préalable à identifier une ou plusieurs images (16_{X}) aux rayons X dans la base (20) locale de données, qui sont similaires à l'image (15) préliminaire ; et
- un moyen pour envoyer à un utilisateur une image (16_{X}) aux rayons X identifiée et ses paramètres (16_{AP}) d'acquisition.

10. Un système de traitement de données suivant la revendication précédente, dans lequel la base (20) locale de données est partagée avec un nombre d'autres systèmes de traitement de données.

11. Un système de traitement de données suivant la revendication 9 ou la revendication 10, configuré pour augmenter la base (20) locale de données par une image (17) prise en utilisant les paramètres (16_{AP}) d'acquisition proposés.

12. Une installation (1) d'imagerie par rayons X comprenant
- une source (10S) de rayons X et un détecteur (11D) de rayons X ;
- un dispositif (14, 10) d'imagerie propre à capter une image (15) préliminaire de la région du corps d'un patient (P), dont on doit prendre une image dans une tâche (34) envisagée d'imagerie par rayons X ;
- une unité (12) de commande propre à commander au moins la source (10S) de rayons X et à recevoir des images (17) du détecteur (11D) de rayons X ; et
- un système (2) de traitement de données suivant l'une quelconque des revendications 9 à 11 propre à recevoir l'image (15) préliminaire et à proposer des paramètres (16_{AP}) d'acquisition sur la base de l'image (15) préliminaire.

13. Une installation d'imagerie par rayons X suivant la revendication précédente, dans lequel le dispositif (14) d'imagerie est l'un quelconque de : un appareil (14) photographique RGB, l'installation (1) par rayons X, un dispositif d'imagerie térahertz, un appareil photographique à champ profond.

14. Un produit de programme d'ordinateur comprenant des instructions qui, lorsque le programme est exécuté par un système (2) de traitement de données suivant l'une quelconque des revendications 9 à 11, font que le système (2) de traitement de données effectue les stades du procédé suivant l'une quelconque des revendications 1 à 5.

15. Un support de données déchiffrable par ordinateur sur lequel est mis en mémoire le produit de programme d'ordinateur de la revendication 14.
